# EUROPEAN PATENT APPLICATION

(11) **EP 4 193 987 A1**
(43) Date of publication of application: **14.06.2023**
(21) Application number: 21852199.5
(22) Date of filing: 06.08.2021
(51) Int. Cl.: A61K 9/00, A61K 9/20, A61K 31/41, A61P 25/00

(54) **SOLID ORAL COMPOSITION COMPRISING CARBAMATE COMPOUND, AND PREPARATION METHOD THEREFOR**

(30) Priority: 06.08.2020 KR 20200098589
(71) Applicant: SK Biopharmaceuticals Co., Ltd., Seongnam-si, Gyeonggi-do 13494 (KR)
(72) Inventor: LEE, Ji Hye, Seongnam-si Gyeonggi-do 13494 (KR); CHOI, So Young, Seongnam-si Gyeonggi-do 13494 (KR); KIM, Sang Jin, Seongnam-si Gyeonggi-do 13494 (KR); YANG, Yun Hee, Seongnam-si Gyeonggi-do 13494 (KR)
(74) Representative: Vossius & Partner Patentanwälte Rechtsanwälte mbB
(86) International application number: PCT/KR2021/010391
(87) International publication number: WO 2022/031099

(57) **Abstract**

The present application relates to a solid oral preparation and a preparation method therefor, the preparation comprising granules, which comprise: as an active ingredient, a carbamate compound of chemical formula 1, or a pharmaceutically acceptable salt, an isomer, solvate or a hydrate thereof; a diluent; and a binder.

## Description

### TECHNICAL FIELD

The present application relates to an oral solid dosage form which comprises granules comprising a carbamate compound of the following Formula 1, or a pharmaceutically acceptable salt, isomer, solvate or hydrate thereof as an active ingredient; a diluent; and a binder; and a preparation method therefor: 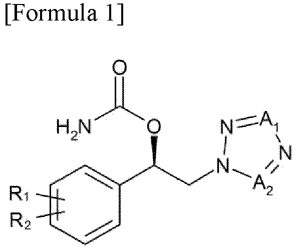 wherein,
R₁, R₂, A₁ and A₂ are the same as defined herein.

### BACKGROUND ART

Central nervous system diseases are a group of neurological disorders that affect the structure or function of the brain or spinal cord that consist of the central nervous system. Causes of central nervous system diseases include trauma, infection, degeneration, structural defects, central nervous system tumors, autoimmune diseases, stroke and the like.

The carbamate compound (carbamic acid aryl-2-tetrazolyl ethyl ester) represented by the following Formula 1 and the preparation method therefor are described in detail in International Patent Publication Nos. WO 2006/112685 A1, WO 2010/150946 A1 and WO 2011/046380 A2, which are incorporated herein by reference: 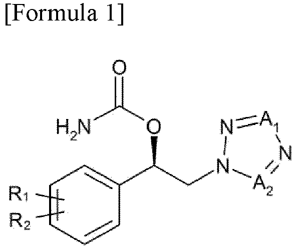 wherein R₁ and R₂ are each independently selected from the group consisting of hydrogen, halogen, C₁-C₈ perfluoroalkyl, C₁-C₈ alkyl, C₁-C₈ thioalkoxy and C₁-C₈ alkoxy; and one of A₁ and A₂ is CH, and the other is N.

A specific example of the carbamate compound of Formula 1 may be a carbamate compound of the following Formula 2 (carbamic acid (R)-1-(2-chlorophenyl)-2-tetrazol-2-yl-ethyl ester):

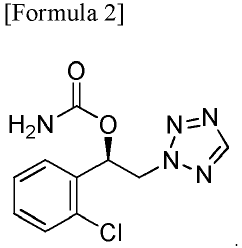

The carbamate compound of Formula 2 is known as an effective anti-epileptic drug used for central nervous system diseases, but studies about a specific formulation for oral administration for applying it to the human body have not been disclosed. In order for drugs to be applied to the human body, formulation design is essential. In order to be effective as drugs, specific formulations such as tablets, capsules, injections and ointments are required.

In the case of obtaining pharmacological activity by administering a preparation comprising said compound, it is required that the effect should appear quickly, and it must be suitable to secure a uniform concentration of the active ingredient in the blood by repeating administration over an extended treatment period. In order to obtain a quick effect, an injection preparation may be appropriate, but there is a disadvantage in that use is limited due to the route of administration. As such, there is increased demand for developing a novel oral solid dosage form for achieving this purpose. In addition, dysphagia is a common symptom in patients with central nervous system diseases, and it is preferable that the size of the preparation be as small as possible because there may be difficulty in swallowing oral preparations.

Clinical studies of carbamates indicate the need for high doses (200 to 400 mg). For convenience in oral administration, tablet weights exceeding 800 mg are generally not preferred. Since the large number of tablets required per dose may cause compliance problems, it would be commercially desirable to develop a formulation that reduces the number of tablets required per dose so as to contribute to improving compliance in patients.

Therefore, there is a need to develop tablet formulations comprising carbamates that have 1) a suitable size for easy swallowing by the patient, 2) a high carbamate content in a single pill to minimize the number of tablets required per dose, and 3) a pharmacological behavior capable of leading to the desired effect with suitable properties regarding the release of carbamates from the tablet.

Meanwhile, the overall physical properties and manufacturability of low-dose drug formulations are entirely determined by inactive ingredients and excipients in the formulation. This is because the physical properties of excipients used in formulations dominate the properties of tablets rather than the physical properties of active ingredients. However, in the case of high-dose drugs, the effect of the physical properties of pharmaceutically active ingredients on the manufacturability of formulations becomes significant. As a loading amount of drug increase in a single pill, the physicochemical properties of active ingredients dominate remarkably that of powder blends in the tablet manufacturing process. Not all active ingredients can have the appropriate properties related to compressibility needed to obtain high-dose tablets.

Said carbamate compound as an active ingredient is an extremely brittle material with low physical plasticity. This makes it difficult to formulate oral solid dosage forms comprising a high content of the carbamate compound. In order to neutralize the brittle property of the carbamate in the manufacture of tablets, it is possible to try to dilute the carbamate by using large amounts of excipients conventionally having properties of ductility/plasticity, but excessive tablet weight may cause problem of dysphagia. In addition, in order to compress tablets-in which a high content of brittle substances is comprised-with a predetermined good hardness and low friability, high-compressing pressure is required, and the low strain rate increases the rate of voids in tablets, resulting in poor tableting such as capping and lamination. In the present application, there is disclosed an oral solid dosage form comprising a high content of the active ingredient, which can minimize tablet weight, and has excellent hardness, and resistance to capping and friability.

### DISCLOSURE OF INVENTION

### TECHNICAL PROBLEM

Accordingly, one object of the present application is the provision of an oral solid dosage form which comprises the carbamate compound of Formula 1, or a pharmaceutically acceptable salt, isomer, solvate or hydrate thereof as an active ingredient in a high loading amount, and has content uniformity, increased convenience of taking drugs and a rapid dissolution rate, and a preparation method therefor.

In addition, another object of the present application is the provision of an oral solid dosage form comprising a high content of the active ingredient, which not only has an excellent dissolution rate and a minimized tablet weight, but also has improved physical properties required for tablet manufacturing, thereby having good compression properties, a predetermined good hardness and resistance to friability.

### SOLUTION TO PROBLEM

The present disclosure provides an oral solid dosage form which comprises granules comprising a carbamate compound of the following Formula 1, or a pharmaceutically acceptable salt, isomer, solvate or hydrate thereof as an active ingredient; a diluent; and a binder: 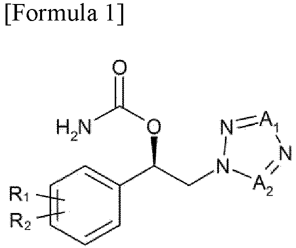 wherein,
R₁ and R₂ are each independently selected from the group consisting of hydrogen, halogen, C₁-C₈ perfluoroalkyl, C₁-C₈ alkyl, C₁-C₈ thioalkoxy and C₁-C₈ alkoxy; and
one of A₁ and A₂ is CH, and the other is N.

In addition, one aspect of the present disclosure provides a method for preparing an oral solid dosage form comprising:
i) blending the carbamate compound of Formula 1, or a pharmaceutically acceptable salt, isomer, solvate or hydrate thereof as an active ingredient with excipients comprising a diluent, a lubricant and a binder, and then compacting;
ii) milling and sieving the compacted product prepared in step (i);
iii) post-blending the granules sieved in step (ii) with excipients comprising a diluent, a lubricant and a disintegrant; and
iv) formulating the granules obtained by post-blending in step (iii).

Furthermore, one aspect of the present disclosure provides a method for preparing an oral solid dosage form comprising:
a) blending the carbamate compound of Formula 1, or a pharmaceutically acceptable salt, isomer, solvate or hydrate thereof as an active ingredient with excipients comprising a diluent and a binder, and a solvent;
b) granulating the mixture obtained in step (a) and then drying and sieving sequentially;
c) post-blending the dried product obtained in step (b) with excipients comprising a lubricant and a disintegrant; and
d) formulating the granules obtained by post-blending in step (c).

According to one embodiment of the present disclosure, there is provided an oral solid dosage form which satisfies content uniformity and dissolution rate without micronizing the active ingredient and has an appropriate size for taking it.

Dysphagia is a common symptom in patients with central nervous system diseases, and it is preferable to adjust the size of oral solid dosage form appropriate for ingestion as possible because there may be difficulty in swallowing oral preparations. In the case of preparing a tablet having a high content of the compound of Formula 1 as an active ingredient by direct compression, in order to increase the density and improve the flowability of the powder, the loading amount of excipients must be significantly increased (e.g., ≥ 75% (w/w)), and in this case the size of the tablet may be increased. Therefore, in the case of a preparation comprising a high content of the compound of Formula 1, greater difficulty is encountered in taking it.

In general, micronization of particles of active ingredients is not adopted since it may decrease the flowability and stability of drugs, thereby affecting the content uniformity and assay. That is, when capsules or tablets are prepared by micronizing the carbamate compound of Formula 1, some problems may be expected that the production cost is increased due to a decrease in yield caused by static electricity, and flowability is bad due to a low density of 0.50 g/mL or less. However, when capsules or tablets comprising a high content of the non-micronized carbamate compound of Formula 1 are prepared, problems of content non-uniformity and low dissolution rate due to the large particle size of the active ingredient may be expected. One embodiment of the present disclosure provides a dosage form which can satisfy the requirements for content uniformity and dissolution rate regardless of whether the main component is micronized or not.

According to one aspect of the present disclosure, a high loading amount (e.g., > 25% (w/w), ≥ 50% (w/w) or ≥ 60% (w/w) in a composition) of the carbamate compound of Formula 1 in a single formulation can be comprised by wet granulation of the carbamate compound of Formula 1. In addition, when preparing granules by wet granulation, it is preferable to use the micronized carbamate compound of Formula 1 in order to improve the content uniformity and dissolution rate of the preparation.

According to another aspect of the present disclosure, a high loading amount (e.g., > 25% (w/w), ≥ 50% (w/w) or ≥ 60% (w/w) in a composition) of the carbamate compound of Formula 1 in a single formulation can be comprised by dry granulation of the carbamate compound of Formula 1. In the case of the dry granulation method, there are advantages that it is possible to prepare at low cost with a simpler process because there is no need for wetting and drying steps according to the use of a binder solution, and it can be processed even in the case of unstable active ingredients that are sensitive to moisture or heat. However, a dry granulation method is not suitable for all active ingredients, and there are limitations to active ingredients that can be applied. In one embodiment of the present disclosure, when an oral solid dosage form is prepared by dry granulation method, the active ingredient may be micronized or not. Specifically, by preparing an oral solid dosage form through dry granulating the carbamate compound of Formula 1 having a large particle size without a micronization process, it can be possible to achieve not only patient compliance by making an oral solid dosage form loading high amount of active ingredient but also acceptable content uniformity and rapid dissolution.

Therefore, granulation of the carbamate compound of Formula 1 according to the present disclosure allows for a high loading amount (e.g., > 25% (w/w), > 50% (w/w) or ≥ 60% (w/w) in a composition) of the carbamate compound of Formula 1 to be comprised in a single formulation, and the size of the formulation can be adjusted.

Meanwhile, the present disclosure provides an oral solid dosage form containing a high content of the active ingredient, which has improved physical properties necessary for tablet manufacturing while minimizing a tablet weight and at the same time has an excellent dissolution rate. The oral solid dosage form according to the present disclosure is not affected by the particle size of carbamate by granulating a preparation comprising a relatively high content of the carbamate compound of Formula 1, and furthermore, it can provide improved compressibility in terms of acceptable hardness and resistance to capping. Specifically, even if the granulation process is carried out by using the carbamate compound of Formula 1 in which the particle size is not adjusted, good tableting and dissolution properties can be provided.

As one concrete embodiment, there is provided an oral solid dosage form, which further comprises a disintegrant in the granules, and further comprises a disintegrant and a lubricant outside the granules.

As one concrete embodiment, there is provided a method for preparing an oral solid dosage form comprising:
I) blending the carbamate compound of Formula 1, or a pharmaceutically acceptable salt, isomer, solvate or hydrate thereof as an active ingredient with excipients comprising a diluent, a lubricant, a binder and a disintegrant, and then compacting;
II) milling and sieving the mixture obtained in step (I) by screening through a sieve;
III) post-blending the granules sieved in step (II) with excipients comprising a disintegrant and a lubricant; and
IV) formulating the granules obtained by post-blending in step (III).

### EFFECTS OF INVENTION

According to the present disclosure, it is possible to provide an oral solid dosage form which shows rapid and consistent therapeutic effects by achieving excellent disintegration, rapid dissolution rate and content uniformity.

According to one embodiment of the present disclosure, it is possible to provide an oral solid dosage form which satisfies content uniformity and dissolution rate without micronizing the active ingredient and has an appropriate size of oral solid dosage form for taking it. In addition, according to one embodiment of the present disclosure, it is possible to provide an oral solid dosage form which comprises a high content of the compound of Formula 1 and also has an appropriate size of oral solid dosage form that is not inconvenient for ingestion by lowering the content of excipients. Furthermore, according to one embodiment of the present disclosure, it is possible to provide an oral solid dosage form which has improved compressibility in terms of acceptable hardness and resistance to capping.

### BRIEF DESCRIPTION OF DRAWINGS

Figure 1 is a graph showing the dissolution test results of Examples 1 to 8.
Figure 2 is a graph showing the dissolution test results of Comparative Examples 1 and 2.
Figure 3 is a graph showing the dissolution test results of Examples 9 and 10.

### MODE FOR THE INVENTION

The present disclosure is described in detail hereinafter.

All technical terms used in the present disclosure, unless otherwise defined, have the same meaning as commonly understood by a person skilled in the art of the present disclosure. In addition, although preferred methods and samples are described herein, similar or equivalent ones are also included within the scope of the present disclosure.

According to one aspect of the present disclosure, there is provided an oral solid dosage form which comprises granules comprising a carbamate compound of the following Formula 1, or a pharmaceutically acceptable salt, isomer, solvate or hydrate thereof as an active ingredient; a diluent; and a binder: 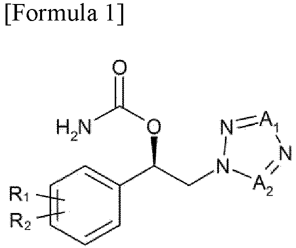 wherein,
R₁ and R₂ are each independently selected from the group consisting of hydrogen, halogen, C₁-C₈ perfluoroalkyl, C₁-C₈ alkyl, C₁-C₈ thioalkoxy and C₁-C₈ alkoxy; and
one of A₁ and A₂ is CH, and the other is N.

The constant dissolution rate of formulations is a necessary condition for rapid and consistent therapeutic effect and quality control, and if the dissolution rate of active ingredients is variable, it may cause problems in quality control of formulations. Depending on drugs, there is a case where the dissolution rate is variable despite high water solubility in the *in vivo* pH range (pH 1.2-6.8). The present disclosure provides an oral solid dosage form comprising the compound of Formula 1 exhibiting a constant dissolution rate in the *in vivo* pH range (pH 1.2-6.8).

Conventional oral solid dosage forms can be prepared by a direct compression method in which all the ingredients are mixed and directly compressed into tablets; a dry granulation method in which the mixture is compacted and milled to prepare dry granules, and then they are compressed into tablets; and a wet granulation method in which a binder solution prepared by using water or an organic solvent is added to a mixture, and kneading, granulation, drying and sizing processes are carried out to prepare wet granules, and then they are compressed into tablets.

In one embodiment of the present disclosure, the oral solid dosage form may comprise 15 to 90% by weight of the active ingredient (the carbamate compound of Formula 1, or a pharmaceutically acceptable salt, isomer, solvate or hydrate thereof). In another embodiment of the present disclosure, the oral solid dosage form may comprise 25 to 65% by weight of the active ingredient. In another embodiment of the present disclosure, the oral solid dosage form may comprise 15 to 70% by weight or 20 to 65% by weight of the active ingredient. In another embodiment of the present disclosure, the oral solid dosage form may comprise about 50% by weight or more-for example, about 50 to 70% by weight of the active ingredient. In another embodiment of the present disclosure, the oral solid dosage form may comprise about 55% by weight or more-for example, about 55 to 70% by weight of the active ingredient.

In another embodiment of the present disclosure, the particle diameter d(0.9) of the active ingredient may be 35 µm to 1,300 µm. In another embodiment of the present disclosure, the active ingredient may be micronized or not, and when the active ingredient is micronized, the particle diameter d(0.9) may be 300 µm or less. More specifically, the particle diameter d(0.9) may have a particle size distribution of 250 µm or less, 200 µm or less, 150 µm or less, 130 µm or less, or 100 µm or less. The lower limit of the particle diameter d(0.9) is not particularly limited, and may be, for example, greater than 0 µm, 30 µm or more, or 50 µm or more, but is not limited thereto. When the active ingredient is not micronized, the particle diameter d(0.9) of the active ingredient may be 300 µm to 1,300 µm. Specifically, the particle diameter d(0.9) of the active ingredient may be 600 µm to 1,000 µm, more specifically 700 µm to 1,000 µm, and more specifically 800 µm to 900 µm.

As used herein, "d(0.9)" means that 90% of the particle volume has a diameter in a specific diameter d range. Specifically, it means that the particle diameter d(0.9) of the point where the cumulative frequency of the volume distribution reaches 90% by accumulating from the particle of the smaller particle diameter is within the range of the specific diameter d.

In another embodiment of the present disclosure, the particle diameter d(0.9) of the active ingredient may be 86 µm to 460 µm. In this case, the active ingredient may be micronized, and a formulation may be prepared by applying a wet granulation method or dry granulation method.

In addition, in another embodiment of the present disclosure, the particle diameter d(0.9) of the active ingredient may be 300 µm to 1,300 µm. In this case, the active ingredient may not be micronized, and a formulation may be prepared by applying a dry granulation method.

It should be understood that all numerical designations-for example, pH, temperature, time, concentration and d(0.9)-used throughout the instant specification may be modified by the term "about" in all instances. When the term "about" is used in the description of the present disclosure, it may mean ±10%, ±5%, ±2% or ±1% in reference to a percentage. In one embodiment, it may mean ±5%, ±2% or ±1%. For example, "about 5" is meant to include any value between 4.5 and 5.5, between 4.75 and 5.25, between 4.9 and 5.1, or between 4.95 and 5.05.

As used herein, the term "particle" refers to individual drug substance particles, whether the particles exist alone or aggregated. That is, the oral solid dosage form of the present disclosure comprising the carbamate compound of Formula 1 may contain aggregates having a particle diameter d(0.9) outside the above numerical range. However, in the case in which the particle diameter d(0.9) of the main drug particles constituting the aggregate is within the above numerical range, it is considered that the aggregate itself satisfies the particle size constraints defined herein, and is within the scope of the present disclosure.

Herein, with respect to the particles of the carbamate compound of Formula 1, reference to particle size such as particle diameter d(0.9) means that the average of particles of the carbamate compound of Formula 1 in the sample has an estimated volume less than or equal to the volume calculated for spherical particles with a diameter equal to a given diameter, based on the assumption that the shape of the particles is spherical. The particle size distribution would be well known to those skilled in the art and can be measured by a laser light scattering technique such as that disclosed and discussed below. In one embodiment of the present disclosure, the particle size of the carbamate compound of Formula 1 was measured by the particle size analyzer distributed by Malvern.

In another embodiment of the present disclosure, the oral solid dosage form may be a tablet dosage form. In another embodiment of the present disclosure, the tablet may comprise the carbamate compound of Formula 1, or a pharmaceutically acceptable salt, isomer, solvate or hydrate thereof; a diluent and a binder; and may be prepared by using a dry granulation method or wet granulation method.

In another embodiment of the present disclosure, the granules may have a density of 0.5 g/mL to 1.5 g/mL, specifically 0.8 g/mL to 1.2 g/mL. In addition, the granules may have a particle size in which 50% or more, specifically 60% or more remains in a 30 to 80 mesh (mesh: according to standard KSA5101-1). In addition, the granules may have a hardness of 1 to 5 kp, specifically may have a hardness of 2 to 3 kp.

In another embodiment of the present disclosure, the oral solid dosage form may further comprise a diluent outside the granules. In another embodiment of the present disclosure, the example of the diluent may be one or more selected from the group consisting of corn starch, pre-gelatinized starch, potato starch, wheat starch, sweet potato starch, tapioca starch, rice starch, beeswax, sucrose, anhydrous lactose, lactose monohydrate, mannitol, sorbitol, xylitol, lactitol, maltitol, erythritol, aluminum silicate, hydroxypropyl starch, microcrystalline cellulose, crystalline cellulose and silicified microcrystalline cellulose, but is not limited thereto. In another embodiment of the present disclosure, the diluent comprised in the granules and the diluent further comprised outside the granules may be the same or different.

In another embodiment of the present disclosure, the diluent may be comprised in an amount of 6 to 75% by weight, 8 to 73% by weight, 10 to 70% by weight, or 15 to 70% by weight based on the total weight of the oral solid dosage form. In another embodiment of the present disclosure, the diluent may be comprised in an amount of 24 to 60% by weight, and preferably 24.07 to 59% by weight based on the total weight of the oral solid dosage form. In another embodiment of the present disclosure, the diluent may be comprised in an amount of 6 to 40% by weight, or 10 to 35% by weight in the granules. In another embodiment of the present disclosure, the diluent may be comprised in an amount of 0 to 50% by weight, or 5 to 50% by weight outside the granules. In another embodiment of the present disclosure, the diluent may be comprised in an amount of 6 to 40% by weight, or 10 to 35% by weight in the granules, and at the same time, in an amount of 0 to 50% by weight, or 5 to 50% by weight outside the granules.

In another embodiment of the present disclosure, the oral solid dosage form may further comprise a lubricant. In another embodiment of the present disclosure, the oral solid dosage form may further comprise the lubricant in the granules, outside the granules, or both. In another embodiment of the present disclosure, the example of the lubricant may be one or more selected from the group consisting of glyceryl behenate, magnesium stearate, mineral oil, polyethylene glycol, polyoxyl 35 castor oil, polyoxyl 40 hydrogenated castor oil, polyoxyl 10 oleyl ether, polyoxyl 20 cetostearyl ether, polyoxyl 40 stearate, polysorbate 20, polysorbate 40, polysorbate 60, polysorbate 80, sodium lauryl sulfate, sodium stearyl fumarate, sorbitan monolaurate, sorbitan monooleate, sorbitan monopalmitate, sorbitan monostearate, sorbitan trioleate, starch, stearic acid, talc and zinc stearate, but is not limited thereto. In another embodiment of the present disclosure, the lubricant comprised in the granules and the lubricant comprised outside the granules may be the same or different.

In another embodiment of the present disclosure, the lubricant may be comprised in an amount of 0.2 to 2% by weight, 0.2 to 1.8% by weight, 0.3 to 1.6% by weight, or 0.3 to 1.5% by weight based on the total weight of the oral solid dosage form. In another embodiment of the present disclosure, the lubricant may be comprised in an amount of 0.4 to 0.7% by weight, and preferably 0.3 to 0.8% by weight based on the total weight of the oral solid dosage form. In another embodiment of the present disclosure, the lubricant may be comprised in an amount of 0.1 to 1% by weight in the granules. In another embodiment of the present disclosure, the lubricant may be comprised in an amount of 0.1 to 2% by weight outside the granules. In another embodiment of the present disclosure, the lubricant may be comprised in an amount of 0.1 to 1% by weight in the granules, and at the same time, in an amount of 0.1 to 2% by weight outside the granules.

In another embodiment of the present disclosure, the example of the binder may be one or more selected from the group consisting of alginic acid, ammonio methacrylate copolymer, ammonio methacrylate copolymer dispersion, carbomer copolymer, carbomer homopolymer, carbomer interpolymer, sodium carboxymethylcellulose, microcrystalline cellulose, copovidone, dextrin, ethyl cellulose, gelatin, liquid glucose, guar gum, low-substituted hydroxypropyl cellulose, hydroxypropyl cellulose, hypromellose, hypromellose acetate succinate, maltodextrin, maltose, methyl cellulose, polyethylene oxide, povidone, corn starch, potato starch, pre-gelatinized starch, modified pre-gelatinized starch and tapioca starch, but is not limited thereto.

In another embodiment of the present disclosure, the binder may be comprised in an amount of 2 to 60% by weight, 2 to 50% by weight, 3 to 40% by weight, 3 to 35% by weight, or 3 to 30% by weight based on the total weight of the oral solid dosage form. In another embodiment of the present disclosure, the binder may be comprised in an amount of 4 to 13% by weight, and specifically 4.47 to 12.5% by weight based on the total weight of the oral solid dosage form.

In another embodiment of the present disclosure, the oral solid dosage form may further comprise a disintegrant. In another embodiment of the present disclosure, the oral solid dosage form may further comprise the disintegrant in the granules, outside the granules, or both. In another embodiment of the present disclosure, the example of the disintegrant may be one or more selected from the group consisting of low-substituted hydroxypropyl cellulose, microcrystalline cellulose, starch, anhydrous lactose, lactose monohydrate, sodium starch glycolate, crospovidone, carboxymethylcellulose and a pharmaceutically acceptable salt thereof, hydroxypropyl cellulose, corn starch and croscarmellose, but is not limited thereto. In another embodiment of the present disclosure, the disintegrant comprised in the granules and the disintegrant comprised outside the granules may be the same or different.

In another embodiment of the present disclosure, the disintegrant may be comprised in an amount of 1 to 15% by weight, 1.5 to 13% by weight, 2 to 12% by weight, or 2 to 10% by weight based on the total weight of the oral solid dosage form. In another embodiment of the present disclosure, the disintegrant may be comprised in an amount of 2 to 3.5% by weight, and preferably 2.35 to 3.01% by weight based on the total weight of the oral solid dosage form. In another embodiment of the present disclosure, the disintegrant may be comprised in an amount of 1 to 10% by weight, or 0.5 to 5% by weight in the granules. In another embodiment of the present disclosure, the disintegrant may be comprised in an amount of 1 to 10% by weight, or 0.5 to 5% by weight outside the granules. In another embodiment of the present disclosure, the disintegrant may be comprised in an amount of 1 to 10% by weight, or 0.5 to 5% by weight in the granules, and at the same time, in an amount of 1 to 10% by weight, or 0.5 to 5% by weight outside the granules.

In another embodiment of the present disclosure, the oral solid dosage form may comprise 15 to 90% by weight of the active ingredient, 6 to 75% by weight of the diluent, 0.2 to 2% by weight of the lubricant, 2 to 50% by weight of the binder and 1 to 15% by weight of the disintegrant. In another embodiment of the present disclosure, the oral solid dosage form may comprise 15 to 70% by weight of the active ingredient, 8 to 73% by weight of the diluent, 0.2 to 1.8% by weight of the lubricant, 3 to 40% by weight of the binder and 1.5 to 13% by weight of the disintegrant. In another embodiment of the present disclosure, the oral solid dosage form may comprise 20 to 65% by weight of the active ingredient, 10 to 70% by weight of the diluent, 0.3 to 1.6% by weight of the lubricant, 4 to 35% by weight of the binder and 2 to 12% by weight of the disintegrant. In another embodiment of the present disclosure, the oral solid dosage form may comprise 25 to 65% by weight of the active ingredient, 15 to 70% by weight of the diluent, 0.3 to 1.5% by weight of the lubricant, 4 to 30% by weight of the binder and 2 to 10% by weight of the disintegrant.

In another embodiment of the present disclosure, the oral solid dosage form may further comprise a glidant. In another embodiment of the present disclosure, the oral solid dosage form may further comprise the glidant in the granules, outside the granules, or both. In another embodiment of the present disclosure, the example of the glidant may be one or more selected from the group consisting of colloidal silicon dioxide, talc, silicon dioxide, light anhydrous silicic acid, aluminum silicate, calcium silicate, calcium hydrogen phosphate and calcium carbonate, but is not limited thereto.

In another embodiment of the present disclosure, the glidant may be comprised in an amount of 0.2 to 2% by weight, 0.2 to 1.8% by weight, 0.3 to 1.6% by weight, or 0.3 to 1.5% by weight based on the total weight of the oral solid dosage form. In another embodiment of the present disclosure, the glidant may be comprised in an amount of 0.2 to 0.4% by weight, and preferably 0.28 to 0.36% by weight based on the total weight of the oral solid dosage form. In another embodiment of the present disclosure, the glidant may be comprised in an amount of 0.1 to 1% by weight in the granules, and at the same time, in an amount of 0.1 to 1% by weight outside the granules.

In another embodiment of the present disclosure, the oral solid dosage form may further comprise a surfactant. In another embodiment of the present disclosure, the oral solid dosage form may further comprise the surfactant in the granules, outside the granules, or both. In another embodiment of the present disclosure, the example of the surfactant may be one or more selected from the group consisting of polysorbate 80, oleoyl macrogolglycerides, caprylocaproyl polyoxylglycerides, linoleoyl polyoxylglycerides, hydroxypropyl methylcellulose, hydroxypropyl cellulose, sodium carboxymethylcellulose, polyvinylpyrrolidone, sodium lauryl sulfate, sodium oleate and sodium dioctylsulfosuccinate, but is not limited thereto.

In another embodiment of the present disclosure, the oral solid dosage form may further comprise a solvent in the granules. The solvent is conventionally used in this technical field and is not specifically limited thereto. In another embodiment of the present disclosure, the solvent may be an organic solvent. In another embodiment of the present disclosure, the solvent may be selected from water, ethanol, isopropanol, methanol, acetone and a combination thereof. In another embodiment of the present disclosure, the solvent is ethanol.

In addition to the above-mentioned ingredients, the oral solid dosage form of the present disclosure may comprise other ingredients such as a film coating agent, a coloring agent, a fragrance, a sweetener, a flavoring agent and a pigment preservative within a range that does not impair the purpose of the oral solid dosage form of the present disclosure. Specifically, these ingredients may be comprised in the granules, outside the granules, or both.

In another embodiment of the present disclosure, the carbamate compound of Formula 1 may be carbamic acid (R)-1-(2-chlorophenyl)-2-tetrazol-2-yl-ethyl ester of the following Formula 2:

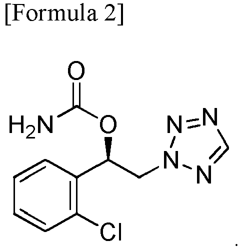

The oral solid dosage form of the present disclosure may be any solid dosage form known in this technical field. Specifically, the solid dosage form may be in the form of tablets or capsules, and more specifically, may be in the form of compressed tablets, multi-compressed tablets, sugar-coated tablets, film-coated tablets, hard capsules or soft capsules. Preferably, the solid dosage form may be in the form of tablets, particularly in the form of compressed tablets or film-coated tablets, but is not limited thereto.

The oral solid dosage form according to the present disclosure may be subjected to a dissolution test according to the United States Pharmacopeia (USP) Apparatus II (paddle method), and preferably exhibits the following dissolution criteria when carrying out an *in vitro* dissolution test. That is, the oral solid dosage form exhibits dissolution characteristics such that more than 80% of the drug is dissolved within 30 minutes, preferably 85% or more, more preferably 90% or more, and still more preferably 91% or more, or 92.5% or more of the drug is dissolved within 30 minutes.

Conventionally, the results of dissolution tests are established as an average over a given number-usually six (6) dosage forms (e.g., tablets, capsules, suspensions or other administration forms). Dissolution tests are typically performed in aqueous medium buffered to the pH range observed in the gastrointestinal tract (pH 1 to 7.4) and adjusted to 37°C (±1°C) to maintain physiologically relevant conditions. When the administration form being tested is a tablet, a paddle rotating at 50 to 75 rpm is typically used to test the dissolution rate of the tablet. The amount of dissolved carbamate compound of Formula 1 can be determined routinely by HPLC. The dissolution test serves as a quality control tool.

In another embodiment of the present disclosure, the oral solid dosage form may comprise the active ingredient (i.e., the carbamate compound of Formula 1, an isomer thereof, or a pharmaceutically acceptable salt, solvate or hydrate thereof) in a dose-range of 5 mg to 600 mg. More specifically, the active ingredient may be comprised in a dose of 12.5 mg, 25 mg, 50 mg, 100 mg, 150 mg, 200 mg, 250 mg, 300 mg, 350 mg, 400 mg, 500 mg, 550 mg or 600 mg.

According to one aspect of the present disclosure, there is provided an oral solid dosage form comprising a high amount of the active ingredient, which not only has an excellent dissolution rate and a minimized tablet weight, but also has improved physical properties required for tablet manufacturing. Specifically, according to the experimental examples of the present disclosure, there is provided a pharmaceutical composition and a preparation method therefor, which has good compressibility, a predetermined hardness, resistance to friability, and good disintegration and dissolution characteristics without occurrence of tableting defects such as capping at the time of manufacturing as a tablet.

In one embodiment of the present disclosure, there is provided an oral solid dosage form, which further comprises a disintegrant in the granules, and further comprises a disintegrant and a lubricant outside the granules.

In one embodiment of the present disclosure, the disintegrant comprised in the granules and the disintegrant comprised outside the granules may be each independently crospovidone; clays such as bentonite, montmorillonite or veegum; cellulose and cellulose derivatives such as microcrystalline cellulose, hydroxypropyl cellulose or carboxymethyl cellulose; algins such as sodium alginate or alginic acid; or a mixture thereof. By comprising the disintegrant, there may be an effect of improving the disintegration rate without gelling. In one embodiment of the present disclosure, the disintegrant comprised in the granules may be comprised in an amount of 0.5 to 5% by weight based on the total weight of the oral solid dosage form, and the disintegrant comprised outside the granules may be comprised in an amount of 0.5 to 5% by weight based on the total weight of the oral solid dosage form.

In one embodiment of the present disclosure, the diluent comprised in the granules may be cellulose derivatives such as microcrystalline cellulose, crystalline cellulose or silicified microcrystalline cellulose, or a mixture thereof. By comprising the diluent, there may be an effect of shortening the disintegration time and improving formulation hardness. In one embodiment of the present disclosure, the diluent comprised in the granules may be comprised in an amount of 6 to 40% by weight, and specifically 5 to 35% by weight based on the total weight of the oral solid dosage form.

In one embodiment of the present disclosure, the diluent may be comprised outside the granules. The diluent comprised outside the granules may be lactose anhydrous. The diluent comprised outside the granules may not be comprised, but the comprisal of the diluent outside the granules may have an effect of improving a formulation hardness. In one embodiment of the present disclosure, the diluent comprised outside the granules may be comprised in an amount of 5 to 50% by weight based on the total weight of the oral solid dosage form.

In one embodiment of the present disclosure, the binder comprised in the granules may be hydroxypropyl cellulose, copovidone or a mixture thereof. By comprising the binder, there may be an effect of increasing a formulation hardness and reducing friability. In one embodiment of the present disclosure, the binder comprised in the granules may be comprised in an amount of 2 to 60% by weight, and specifically 3 to 40% by weight based on the total weight of the oral solid dosage form.

In one embodiment of the present disclosure, the glidant may be comprised outside the granules.

In one embodiment in which physical properties of the present disclosure are improved, the property, size, appearance, type, content, and whether or not the excipients are comprised in or outside the granules, etc., which are not specifically described in detail, follow the above description.

In one embodiment of the present disclosure, the oral solid dosage form may comprise 15 to 90% by weight of active ingredient, 6 to 40% by weight of diluent in granules, 0.1 to 1% by weight of lubricant in granules, 2 to 60% by weight of binder in granules, 0.5 to 5% by weight of disintegrant in granules, 0.5 to 5% by weight of disintegrant outside granules, and 0.1 to 2% by weight of lubricant outside granules. In one embodiment of the present disclosure, the oral solid dosage form may comprise 25 to 60% by weight of active ingredient, 5 to 35% by weight of diluent in granules, 0.1 to 1% by weight of lubricant in granules, 3 to 40% by weight of binder in granules, 0.5 to 5% by weight of disintegrant in granules, 0.5 to 5% by weight of disintegrant outside granules, and 0.1 to 2% by weight of lubricant outside granules. In the above embodiment, the diluent comprised in the granules may be microcrystalline cellulose, the binder comprised in the granules may be hydroxypropyl cellulose, and the disintegrant comprised in the granules and disintegrant comprised outside the granules may be crospovidone.

In one embodiment of the present disclosure, the oral solid dosage form may be a tablet formulation. As the tablet formulation, there is no specific limitation. For example, in addition to round shape, the tablet formulation may be other shapes such as elliptical shape, donut shape, etc. The tablet thickness is also not specifically limited, but the tablet thickness may be 1.0 to 10.0 mm, specifically 2.0 to 8.0 mm, more specifically 3.0 to 5.5 mm. The size of the tablet is not specifically limited, but may be, for example, 6 to 20 mm in short diameter (diameter in the case of round tablets), 8.0 to 12.0 mm, and more specifically 9.0 to 10.5 mm.

According to one experimental example of the present disclosure, the oral solid dosage form described above has excellent hardness, friability and resistance to capping. The hardness of oral solid dosage form can be measured by a hardness test according to the United States Pharmacopeia (USP) Monograph <1217>. Tablets must have sufficient mechanical strength to prevent damage during subsequent processing or transport. The mechanical strength of a tablet can be measured by a hardness test, which is related to the size of the tablet. When tablet hardness is measured in kilo-pounds (kp), it is usually 0.8 × tablet diameter (mm) or more, more preferably 1.1 × tablet diameter or more, specifically 1.2 × tablet diameter or more, and specifically 1.3 × tablet diameter or more. The higher the hardness, the stronger the tablet, so that the aforementioned damage during subsequent processing and transportation of tablets can be prevented. However, in the case of very high level of hardness, there may be a problem that the disintegration time of tablets is excessively long. Therefore, it is desirable to prepare tablets by selecting a hardness that avoids the above-mentioned damage and at the same time shows an appropriate disintegration time of the tablet. According to one experimental example of the present disclosure, the above-mentioned oral solid dosage form may have a hardness of 8 to 20 kp, and specifically may have a hardness of 8.5 to 16 kp.

Capping refers to the complete or partial separation of the dish-shaped disk from the upper or lower surface of the tablet during compression of the material to form the tablet or during subsequent processing and/or treatment. Friction refers to a phenomenon in which the tablet surface is damaged, or cracks or fractures appear during compression or subsequent processing and/or treatment, and friability refers to the physical strength of the tablet by quantifying the degree of this phenomenon. Since the capping and friction phenomena can occur when the hardness of the tablet is too weak, as a means to prevent this phenomenon, it is preferable to prepare tablets by selecting the appropriate hardness of the tablet so that the capping phenomenon does not occur and at the same time the tablet has a friability of less than 1% of the mass. According to one experimental example of the present disclosure, the above-mentioned oral solid dosage form has a friability of 1.0% or less, specifically a friability of 0.5% or less, more specifically a friability of 0.3% or less, and more specifically a friability of 0.1 to 0.3%.

In another embodiment of the present disclosure, when the oral solid dosage form is in the form of a film-coated tablet, the oral solid dosage form may comprise a film coating agent. Typically, the film coating agent may be comprised in an amount of 2 to 4% by weight based on the total weight of the oral solid dosage form, and which includes a film-forming agent, a plasticizer, a lubricant and optionally one or more pigments. In another embodiment of the present disclosure, the film-coated tablet may be prepared by additionally carrying out a coating step after tableting. As the film coating agent, a conventional film coating agent such as Opadry may be used.

According to another aspect to the present disclosure, there is provided a method for preparing an oral solid dosage form comprising:
i) blending the carbamate compound of Formula 1, or a pharmaceutically acceptable salt, isomer, solvate or hydrate thereof as an active ingredient with excipients comprising a diluent, a lubricant and a binder, and then compacting;
ii) milling and sieving the compacted product prepared in step (i);
iii) post-blending the granules sieved in step (ii) with excipients comprising a diluent, a lubricant and a disintegrant; and
iv) formulating the granules obtained by post-blending in step (iii).

In another embodiment of the present disclosure, the step (i) may be carried out by compacting a mixture comprising an active ingredient, a diluent, a lubricant and a binder by a roller compaction method or slugging method. In another embodiment of the present disclosure, the step (i) may be carried out by using a roller compaction method. Roller compaction refers to a method of preparing granules through a method of compacting at a constant pressure while passing the powder between two rollers. In another embodiment of the present disclosure, the roller compaction method may be carried out by using a roller compactor. In another embodiment of the present disclosure, the roller-compacted mixture may then undergo a process of milling by using a fitz mill, oscillator or the like, and sieving to obtain granules having an appropriate size, if necessary.

In another embodiment of the present disclosure, the active ingredient of step (i) may be micronized or not. In another embodiment of the present disclosure, the particle diameter d(0.9) of the micronized active ingredient in step (i) may be 300 µm or less, and the particle diameter d(0.9) of the non-micronized active ingredient may be 300 µm to 1,300 µm.

In another embodiment of the present disclosure, micronization of the active ingredient may be carried out by milling. Specifically, the micronization may be carried out by a method using tumbler mills such as ball mills, a method using fluid energy mills such as jet mills, a method using impact mills such as hammer mills and pin mills, but is not limited thereto.

According to another aspect of the present disclosure, there is provided a method for preparing an oral solid dosage form comprising:
a) blending the carbamate compound of Formula 1, or a pharmaceutically acceptable salt, isomer, solvate or hydrate thereof as an active ingredient with excipients comprising a diluent and a binder, and a solvent;
b) granulating the mixture obtained in step (a) and then drying and sieving sequentially;
c) post-blending the dried product obtained in step (b) with excipients comprising a lubricant and a disintegrant; and
d) formulating the granules obtained by post-blending in step (c).

In another embodiment of the present disclosure, the active ingredient of step (a) may be micronized, and specifically, the particle diameter d(0.9) of the micronized active ingredient may be 300 µm or less.

In another embodiment of the present disclosure, the order of blending in step (a) is not limited. For example, the remaining ingredients may be added to a binder solution in which a solvent and a binder are mixed. In addition, the other ingredients including a binder may be added to a solvent. In another embodiment of the present disclosure, the solvent may be an organic solvent. In another embodiment of the present disclosure, the solvent may be selected from water, ethanol, isopropanol, methanol, acetone and a combination thereof. In another embodiment of the present disclosure, the solvent is ethanol. The solvent may be comprised within the allowable range defined by the ICH guidelines after preparation of the formulation.

In another embodiment of the present disclosure, the solvent drying process in step (b) may be carried out at a temperature not exceeding about 70°C, preferably at a temperature not exceeding about 60°C, and more preferably at a temperature of 20°C to 50°C, considering the stability of the active ingredient by air drying, fluidized bed drying, oven drying or microwave drying.

According to one aspect of the present disclosure related to the solid dosage form with improved physical properties described above, there is provided a method for preparing an oral solid dosage form comprising:
I) blending the carbamate compound of Formula 1, or a pharmaceutically acceptable salt, isomer, solvate or hydrate thereof as an active ingredient with excipients comprising a diluent, a lubricant, a binder and a disintegrant, and then compacting;
II) milling and sieving the mixture obtained in step (I) by screening through a sieve;
III) post-blending the granules sieved in step (II) with excipients comprising a disintegrant and a lubricant; and
IV) formulating the granules obtained by post-blending in step (III).

In another embodiment of the present disclosure, the step (I) may be carried out by compacting a mixture comprising an active ingredient, a diluent, a lubricant, a binder and a disintegrant by a roller compaction method or slugging method. In another embodiment of the present disclosure, the step (I) may be carried out by using a slugging method. In detail, the slugging method refers to a method of preparing granules in the form of wide and flat tablets or pellets by pounding or compacting the mixture powder by using a single punch tablet press or the like. In another embodiment of the present disclosure, the slugging method may be carried out by using a single punch tablet press. In another embodiment of the present disclosure, the prepared compacting product may be then screened using a sieve, or undergone a process of milling by using a fitz mill, oscillator or the like, and sieving to obtain granules having an appropriate size, if necessary.

In another embodiment of the present disclosure, the compacted product prepared in step (I) may have a density of 0.5 to 1.5 g/mL, specifically 0.8 to 1.2 g/mL. In addition, the compacted product prepared in step (I) may have a hardness of 1 to 5 kp, specifically a hardness of 2 to 3 kp.

The above-described oral solid dosage form may be prepared by the methods for preparing an oral solid dosage form according to the embodiments of the present disclosure. Therefore, all explanations about the nature, size, appearance, type, content, and whether they are comprised in or outside the granules of diluent, binder, disintegrant, lubricant, glidant and other ingredients described above are shared in those used in the methods for preparing an oral solid dosage form.

The oral solid dosage form prepared by the above method may be the shape, for example, round, oval, oblong, rectangular, cylindrical or other suitable shape. In addition, the size may be changed according to the content of the active ingredient.

In another embodiment of the present disclosure, when the oral solid dosage form is in the form of a coated tablet, the step of coating after tableting may be further comprised. Specifically, the coated tablet may be a film-coated tablet. Typically, the film-coating agent may be comprised in an amount of 2% by weight to 4% by weight based on the total weight of the oral solid dosage form. The film coating agent may include a film-forming agent, a plasticizer, a lubricant and optionally one or more pigments. As the film-coating agent, a conventional film coating agent such as Opadry may be used.

The oral solid dosage form provided according to the present disclosure can be used for preventing or treating diseases of the central nervous system.

In another embodiment of the present disclosure, the central nervous system disease may be selected from anxiety, depression, convulsion, epilepsy, migraine, bipolar disorder, drug abuse, smoking, attention deficit hyperactivity disorder (ADHD), obesity, sleep disorders, stroke, neuropathic pain, cognitive disorders, neurodegeneration and muscle spasm, but is not limited thereto.

As used herein, the terms "prevent," "preventing" and "prevention" refer to reducing or eliminating the likelihood of a disease.

As used herein, the terms "treat," "treating" and "treatment" refer to eliminating a disease and/or its accompanying symptoms altogether or in part.

Hereinafter, the present disclosure will be explained in more detail through examples. However, the following examples are only intended to illustrate one or more embodiments and are not intended to limit the scope of the disclosure.

### Examples 1 to 7

Solid granules were prepared using the ingredients and contents represented in Table 1, and the preparation method is as follows. After blending carbamic acid (R)-1-(2-chlorophenyl)-2-tetrazol-2-yl-ethyl ester of Formula 2 with hydroxypropyl methylcellulose and microcrystalline cellulose, magnesium stearate was added thereto and mixed. Then, the mixture was compacted with a force of 3,000 to 3,500 LBF in a roller compactor (Freund Corporation, TFC-Lab) to prepare a plate-shaped compressed product, and then granules were prepared by milling and sieving in an oscillator (Freund Corporation, vector screen - 20 mesh). The granules were mixed with the diluent, glidant and disintegrant listed in Table 1, and then magnesium stearate was additionally added as a lubricant, and then tableted.

**[Table 1]**

| **Item** | | **Example 1** | | **Example 2** | | **Example 3** | | **Example 4** | | **Example 5** | | **Example 6** | | **Example 7** | |
|---|---|---|---|---|---|---|---|---|---|---|---|---|---|---|---|
| Ingredient | | Weight per tablet (mg) | % by weight | Weight per tablet (mg) | % by weight | Weight per tablet (mg) | % by weight | Weight per tablet (mg) | % by weight | Weight per tablet (mg) | % by weight | Weight per tablet (mg) | % by weight | Weight per tablet (mg) | % by weight |
| First blending | Carbamate compound of Formula 2 | 200.0 | 64.10 | 200.0 | 50.00 | 200.0 | 64.10 | 200.0 | 64.10 | 200.0 | 64.10 | 50.0 | 25.0 | 400.0 | 59.63 |
| | Particle diameter d(0.9) | <460 µm> | | <460 µm> | | <460 µm> | | <865 µm> | | <99 µm> | | <460 µm> | | <865 µm> | |
| | Microcrystalline cellulose (diluent) | 25.0 | 8.01 | 25.5 | 6.38 | 35.0 | 11.22 | 25.0 | 8.01 | 25.0 | 8.01 | 35.0 | 17.50 | 68.4 | 10.20 |
| | Magnesium stearate (lubricant) | 0.8 | 0.26 | 0.8 | 0.20 | 0.8 | 0.26 | 0.8 | 0.26 | 0.8 | 0.26 | 0.8 | 0.40 | 1.6 | 0.24 |
| | Hydroxypropyl methylcellulose (binder) | 25.0 | 8.01 | 25.0 | 6.25 | 150 | 4.81 | 25.0 | 8.01 | 25.0 | 8.01 | 25.0 | 12.50 | 30.0 | 4.47 |
| Sieving | | 20 mesh | | 20 mesh | | 20 mesh | | 20 mesh | | 20 mesh | | 20 mesh | | 20 mesh | |
| Second blending | Lactose monohydrate (diluent) | 50.1 | 16.06 | 49.9 | 12.48 | 50.1 | 16.06 | 50.1 | 16.06 | 50.1 | 16.06 | 65.1 | 32.55 | 104.0 | 15.50 |
| | Microcrystalline cellulose (diluent) | - | - | 87.5 | 21.88 | - | - | - | - | - | - | 17.9 | 8.95 | 43.6 | 6.50 |
| | Magnesium stearate (lubricant) | 0.8 | 0.26 | 0.8 | 0.20 | 0.8 | 0.26 | 0.8 | 0.26 | 0.8 | 0.26 | 0.6 | 0.30 | 2.0 | 0.30 |
| | Silicon dioxide (glidant) | 0.9 | 0.29 | 1.1 | 0.28 | 0.9 | 0.29 | 0.9 | 0.29 | 0.9 | 0.29 | 0.6 | 0.30 | 2.4 | 0.36 |
| | Sodium starch glycolate (disintegrant) | 9.4 | 3.01 | 9.4 | 2.35 | 9.4 | 3.01 | 9.4 | 3.01 | 9.4 | 3.01 | 5.0 | 2.50 | 18.8 | 2.80 |
| Total weight | | 312.0 | 100.00 | 400.0 | 100.0 | 312.0 | 100.00 | 312.0 | 100.00 | 312.0 | 100.00 | 200.0 | 100.00 | 670.8 | 100.00 |

### Comparative Examples 1 and 2

According to the composition and content represented in Table 2, the ingredients were mixed and compressed (direct compression).

**[Table 2]**

| **Item** | | **Comparative Example 1** | | **Comparative Example 2** | |
|---|---|---|---|---|---|
| Ingredient | | Weight per tablet (mg) | % by weight | Weight per tablet (mg) | % by weight |
| First blending | Carbamate compound of Formula 2 | 200.0 | 25.00 | 200.0 | 25.00 |
| | Particle diameter d(0.9) | <460 µm> | | <99 µm> | |
| | Colloidal silicon dioxide (glidant) | 2.2 | 0.28 | 2.2 | 0.28 |
| Sieving | | 20 mesh | | 20 mesh | |
| Second blending | Microcrystalline cellulose (diluent) | 345.2 | 43.15 | 345.2 | 43.15 |
| | Lactose monohydrate (diluent) | 217.4 | 27.17 | 217.4 | 27.17 |
| | Sodium starch glycolate (disintegrant) | 30.5 | 3.81 | 30.5 | 3.81 |
| | Magnesium stearate (lubricant) | 4.7 | 0.59 | 4.7 | 0.59 |
| Total weight (mg) | | 800.0 | 100.00 | 800.0 | 100.00 |

### Example 8

According to the content represented in Table 3, the ingredients were mixed in a highspeed mixer, and hydroxypropyl cellulose and ethanol were then added as a binding solution to knead and granulate. The granules were dried in a dryer adjusted at a temperature of 60°C until the loss on drying reached 0.5 to 2%. The resulting dried product was screened by using a sieve for pulverization and sieved. The ingredients (disintegrant, lubricant and glidant) represented in Table 3 were added to the obtained granules and finally mixed using a V-type mixer. The obtained final mixture was compressed into tablets by using a compression device.

**[Table 3]**

| **Item** | | **Example 8** | |
|---|---|---|---|
| Ingredient | | Weight per tablet (mg) | % by weight |
| First blending | Carbamate compound of Formula 2 | 200.0 | 62.40 |
| | Particle diameter d(0.9) | <99 µm> | |
| | Microcrystalline cellulose (diluent) | 57.6 | 17.94 |
| | Dibasic calcium phosphate (diluent) | 28.0 | 8.99 |
| Binding solution | Hydroxypropyl cellulose (binder) | 12.8 | 3.99 |
| | 90% ethanol | 132.0 | - |
| Sieving | | 20 mesh | |
| Second blending | Sodium starch glycolate (disintegrant) | 19.2 | 5.93 |
| | Magnesium stearate (lubricant) | 1.6 | 0.50 |
| | Silicon dioxide (glidant) | 0.8 | 0.25 |
| Total weight | | 320.0 | 100.00 |

### Examples 9 to 12

Additionally, physical properties such as compressibility necessary for preparing low-plasticity carbamate compound into high-content tablets were to be improved. Solid granules were prepared by using the ingredients and contents represented in Table 4, and the preparation method is as follows. After blending carbamic acid (R)-1-(2-chlorophenyl)-2-tetrazol-2-yl-ethyl ester of Formula 2 with hydroxypropyl cellulose and microcrystalline cellulose, sodium stearyl fumarate was added thereto and mixed. Then, the mixture was compacted by using a single punch tablet press (Korsch AG, EK0 single punch tablet press combined with AR402 central motor unit [ERWEKA GmbH]) to prepare a slug having a density of 1.0 to 1.1 g/mL and a hardness of 2 to 3 kp, and granules were prepared by sieving the slug through an 18-mesh sieve. After blending the granules with the glidant and disintegrant listed in Table 4, sodium stearyl fumarate was further added as a lubricant, and then tableted.

**[Table 4]**

| **Item** | | **Example 9** | | **Example 10** | | **Example 11** | | **Example 12** | |
|---|---|---|---|---|---|---|---|---|---|
| Ingredient | | Weight per tablet (mg) | % by weight | Weight per tablet (mg) | % by weight | Weight per tablet (mg) | % by weight | Weight per tablet (mg) | % by weight |
| First blending | Carbamate compound of Formula 2 | 200.0 | 58.82 | 400.0 | 58.82 | 200.0 | 58.82 | 200.0 | 58.82 |
| | Particle diameter d(0.9) | <846 µm> | | <846 µm> | | <846 µm> | | <846 µm> | |
| | Microcrystalline cellulose (diluent) | 71.4 | 21.00 | 142.8 | 21.00 | 102.1 | 30.03 | 105.5 | 31.03 |
| | Sodium stearyl fumarate (lubricant) | 0.9 | 0.26 | 1.8 | 0.26 | 0.9 | 0.26 | 0.9 | 0.26 |
| | Hydroxypropyl cellulose (binder) | 18.7 | 5.50 | 37.4 | 5.50 | 17.0 | 5.00 | 13.6 | 4.00 |
| | Crospovidone (disintegrant) | 11.1 | 3.26 | 22.2 | 3.26 | 8.5 | 2.50 | 8.5 | 2.50 |
| Sieving | | 18 mesh | | 18 mesh | | 18 mesh | | 18 mesh | |
| Second blending | Anhydrous lactose (diluent) | 23.8 | 7.00 | 47.6 | 7.00 | - | - | - | - |
| | Crospovidone (disintegrant) | 11.1 | 3.26 | 22.2 | 3.26 | 8.5 | 2.50 | 8.5 | 2.50 |
| | Sodium stearyl fumarate (lubricant) | 2.5 | 0.74 | 5.0 | 0.74 | 2.5 | 0.74 | 2.5 | 0.74 |
| | Colloidal silicon dioxide (glidant) | 0.5 | 0.15 | 1.0 | 0.15 | 0.5 | 0.15 | 0.5 | 0.15 |
| Total weight | | 340.0 | 100.00 | 680.0 | 100.0 | 340.0 | 100.00 | 340.0 | 100.00 |

### Experimental Example 1: Dissolution test

A dissolution test was performed on the solid dosage forms prepared in Examples 1 to 10 and Comparative Examples 1 and 2 according to the US Pharmacopoeia under the following conditions.

### <Dissolution conditions>

Dissolution media: 0.01 N hydrochloric acid aqueous solution, 900 mL
Device: Apparatus II (paddle method), 75 rpm
Temperature: 37°C

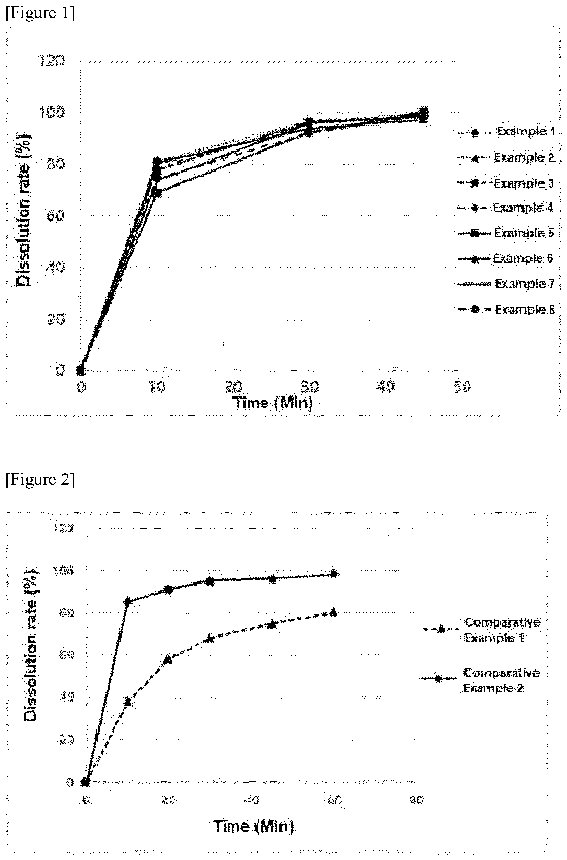

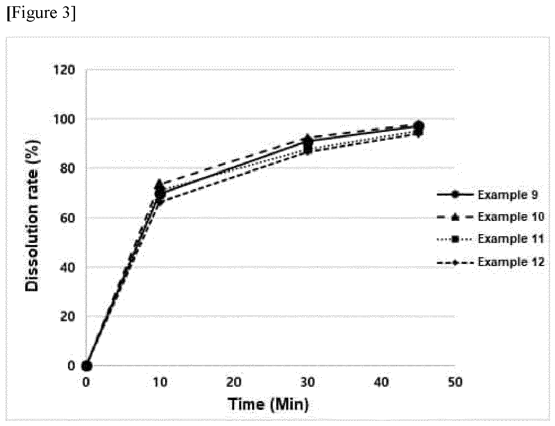

The results of dissolution rate are represented in Tables 5 and 6, and Figures 1 to 3. In Table 5, the dissolution rate was expressed as % of the content of the active ingredient released based on the content of the active ingredient comprised in the tablet.

**[Table 5]**

| | Dissolution rate (%) | | | | | | | | | | | |
|---|---|---|---|---|---|---|---|---|---|---|---|---|
| Time (min) | Example 1 | Example 2 | Example 3 | Example 4 | Example 5 | Example 6 | Example 7 | Example 8 | Example 9 | Example 10 | Example 11 | Example 12 |
| 0 | 0 | 0 | 0 | 0 | 0 | 0 | 0 | 0 | 0 | 0 | 0 | 0 |
| 10 | 81.0 | 77.8 | 77.8 | 78.9 | 69.0 | 80.5 | 73.4 | 74.6 | 69.8 | 73.7 | 71.0 | 66.3 |
| 30 | 96.8 | 96.5 | 96.5 | 96.0 | 92.5 | 94.0 | 96.2 | 92.3 | 91.0 | 92.3 | 87.8 | 86.7 |
| 45 | 99.0 | 99.2 | 99.2 | 99.0 | 100.2 | 97.4 | 98.7 | 99.1 | 97.2 | 97.9 | 95.2 | 94.0 |

**[Table 6]**

| Time (min) | Dissolution rate (%) | |
|---|---|---|
| | Comparative Example 1 | Comparative Example 2 |
| 0 | 0 | 0 |
| 10 | 38 | 79 |
| 20 | 58 | 86 |
| 30 | 68 | 91 |
| 45 | 75 | 95 |
| 60 | 80 | 97 |

### Experimental Example 2: Hardness test

The hardness of the tablets prepared according to Examples 9 to 12 was measured according to the US Pharmacopoeia Monograph <1217>, the friability was measured according to the US Pharmacopoeia Monograph <1216>, the occurrence of capping was checked, and the results are represented in Table 7.

**[Table 7]**

| Item | Example 9 | Example 10 | Example 11 | Example 12 |
|---|---|---|---|---|
| Hardness (kp) | 8.56 | 15.34 | 10.05 | 10.13 |
| Friability (%) | 0.22 | 0.20 | 0.22 | 0.17 |
| Capping occurrence | No | No | No | No |

## Claims

1. An oral solid dosage form which comprises granules comprising a carbamate compound of the following Formula 1, or a pharmaceutically acceptable salt, isomer, solvate or hydrate thereof as an active ingredient; a diluent; and a binder: 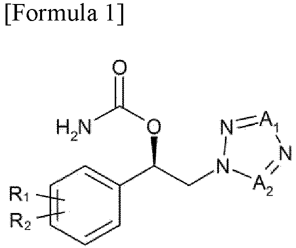 wherein,
R₁ and R₂ are each independently selected from the group consisting of hydrogen, halogen, C₁-C₈ perfluoroalkyl, C₁-C₈ alkyl, C₁-C₈ thioalkoxy and C₁-C₈ alkoxy; and
one of A₁ and A₂ is CH, and the other is N.

2. The oral solid dosage form according to Claim 1, which comprises 15 to 90% by weight of the active ingredient.

3. The oral solid dosage form according to Claim 1 or 2, which comprises 25 to 65% by weight of the active ingredient.

4. The oral solid dosage form according to any one of Claims 1 to 3, wherein the particle diameter d(0.9) of the active ingredient is 35 µm to 1,300 µm.

5. The oral solid dosage form according to any one of Claims 1 to 4, wherein the particle diameter d(0.9) of the active ingredient is 300 µm or less.

6. The oral solid dosage form according to any one of Claims 1 to 5, which further comprises a diluent outside the granules.

7. The oral solid dosage form according to any one of Claims 1 to 6, wherein the diluent is one or more selected from the group consisting of corn starch, pre-gelatinized starch, potato starch, wheat starch, sweet potato starch, tapioca starch, rice starch, beeswax, sucrose, anhydrous lactose, lactose monohydrate, mannitol, sorbitol, xylitol, lactitol, maltitol, erythritol, aluminum silicate, hydroxypropyl starch, microcrystalline cellulose, crystalline cellulose and silicified microcrystalline cellulose.

8. The oral solid dosage form according to any one of Claims 1 to 7, wherein the diluent comprised in the granules is comprised in an amount of 6 to 40% by weight.

9. The oral solid dosage form according to Claim 6, wherein the diluent further comprised outside the granules is comprised in an amount of 5 to 50% by weight.

10. The oral solid dosage form according to any one of Claims 1 to 9, which further comprises a lubricant in the granules, outside the granules, or both.

11. The oral solid dosage form according to Claim 10, wherein the lubricant is one or more selected from the group consisting of glyceryl behenate, magnesium stearate, mineral oil, polyethylene glycol, polyoxyl 35 castor oil, polyoxyl 40 hydrogenated castor oil, polyoxyl 10 oleyl ether, polyoxyl 20 cetostearyl ether, polyoxyl 40 stearate, polysorbate 20, polysorbate 40, polysorbate 60, polysorbate 80, sodium lauryl sulfate, sodium stearyl fumarate, sorbitan monolaurate, sorbitan monooleate, sorbitan monopalmitate, sorbitan monostearate, sorbitan trioleate, starch, stearic acid, talc and zinc stearate.

12. The oral solid dosage form according to Claim 10 or 11, wherein the lubricant comprised in the granules is comprised in an amount of 0.1 to 1% by weight.

13. The oral solid dosage form according to any one of Claims 10 to 12, wherein the lubricant further comprised outside the granules is comprised in an amount of 0.1 to 2% by weight.

14. The oral solid dosage form according to any one of Claims 1 to 13, wherein the binder is one or more selected from the group consisting of alginic acid, ammonio methacrylate copolymer, ammonio methacrylate copolymer dispersion, carbomer copolymer, carbomer homopolymer, carbomer interpolymer, sodium carboxymethylcellulose, microcrystalline cellulose, copovidone, dextrin, ethyl cellulose, gelatin, liquid glucose, guar gum, low-substituted hydroxypropyl cellulose, hydroxypropyl cellulose, hypromellose, hypromellose acetate succinate, maltodextrin, maltose, methyl cellulose, polyethylene oxide, povidone, corn starch, potato starch, pre-gelatinized starch, modified pre-gelatinized starch and tapioca starch.

15. The oral solid dosage form according to any one of Claims 1 to 14, wherein the binder is comprised in an amount of 2 to 60% by weight.

16. The oral solid dosage form according to any one of Claims 1 to 15, which further comprises a disintegrant in the granules, outside the granules, or both.

17. The oral solid dosage form according to Claim 16, wherein the disintegrant is one or more selected from the group consisting of low-substituted hydroxypropyl cellulose, microcrystalline cellulose, starch, anhydrous lactose, lactose monohydrate, sodium starch glycolate, crospovidone, carboxymethylcellulose and a pharmaceutically acceptable salt thereof, hydroxypropyl cellulose, corn starch and croscarmellose.

18. The oral solid dosage form according to Claim 16 or 17, wherein the disintegrant comprised in the granules is comprised in an amount of 1 to 10% by weight.

19. The oral solid dosage form according to any one of Claims 16 to 18, wherein the disintegrant further comprised outside the granules is comprised in an amount of 1 to 10% by weight.

20. The oral solid dosage form according to any one of Claims 1 to 19, which further comprises a glidant in the granules, outside the granules, or both.

21. The oral solid dosage form according to Claim 20, wherein the glidant comprised in the granules is comprised in an amount of 0.1 to 1% by weight.

22. The oral solid dosage form according to Claim 20 or 21, wherein the glidant further comprised outside the granules is comprised in an amount of 0.1 to 1% by weight.

23. The oral solid dosage form according to any one of Claims 1 to 22, which further comprises a solvent in the granules.

24. The oral solid dosage form according to Claim 1, which further comprises a disintegrant in the granules, and further comprises a disintegrant and a lubricant outside the granules.

25. The oral solid dosage form according to Claim 24, wherein the disintegrant comprised in the granules and the disintegrant comprised outside the granules are each independently crospovidone, bentonite, montmorillonite, veegum, microcrystalline cellulose, hydroxypropyl cellulose, carboxymethyl cellulose, sodium alginate, alginic acid or a mixture thereof.

26. The oral solid dosage form according to Claim 24 or 25, wherein the diluent comprised in the granules is microcrystalline cellulose, crystalline cellulose, silicified microcrystalline cellulose or a mixture thereof.

27. The oral solid dosage form according to any one of Claims 24 to 26, wherein the binder comprised in the granules is hydroxypropyl cellulose, copovidone or a mixture thereof.

28. The oral solid dosage form according to any one of Claims 24 to 27, wherein the disintegrant comprised in the granules is comprised in an amount of 0.5 to 5% by weight based on the total weight of the solid preparation for oral administration, and the disintegrant comprised outside the granules is comprised in an amount of 0.5 to 5% by weight based on the total weight of the oral solid dosage form.

29. The oral solid dosage form according to any one of Claims 24 to 28, wherein the diluent comprised in the granules is comprised in an amount of 5 to 50% by weight based on the total weight of the oral solid dosage form.

30. The oral solid dosage form according to any one of Claims 24 to 29, wherein the binder comprised in the granules is comprised in an amount of 2 to 60% by weight based on the total weight of the oral solid dosage form.

31. The oral solid dosage form according to any one of Claims 24 to 30, which has a hardness of 8 to 20 kp.

32. The oral solid dosage form according to any one of Claims 24 to 31, which has a friability of 1.0% or less.

33. The oral solid dosage form according to any one of Claims 1 to 32, wherein the carbamate compound of Formula 1 is carbamic acid (R)-1-(2-chlorophenyl)-2-tetrazol-2-yl-ethyl ester of the following Formula 2:

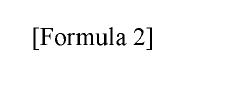

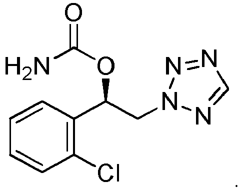

34. A method for preparing an oral solid dosage form comprising:
i) blending a carbamate compound of the following Formula 1, or a pharmaceutically acceptable salt, isomer, solvate or hydrate thereof as an active ingredient with excipients comprising a diluent, a lubricant and a binder, and then compacting;
ii) milling and sieving the compacted product prepared in step (i);
iii) post-blending the granules sieved in step (ii) with excipients comprising a diluent, a lubricant and a disintegrant; and
iv) formulating the granules obtained by post-blending in step (iii): 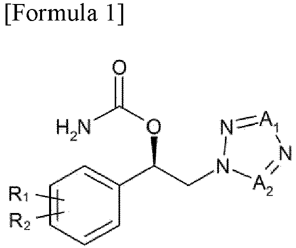 wherein,
R₁ and R₂ are each independently selected from the group consisting of hydrogen, halogen, C₁-C₈ perfluoroalkyl, C₁-C₈ alkyl, C₁-C₈ thioalkoxy and C₁-C₈ alkoxy; and
one of A₁ and A₂ is CH, and the other is N.

35. A method for preparing an oral solid dosage form comprising:
a) blending a carbamate compound of the following Formula 1, or a pharmaceutically acceptable salt, isomer, solvate or hydrate thereof as an active ingredient with excipients comprising a diluent and a binder, and a solvent;
b) granulating the mixture obtained in step (a) and then drying and sieving sequentially;
c) post-blending the dried product obtained in step (b) with excipients comprising a lubricant and a disintegrant; and
d) formulating the granules obtained by post-blending in step (c): 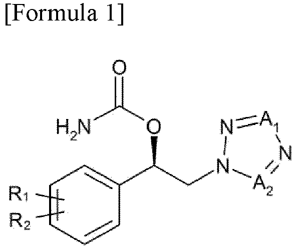 wherein,
R₁ and R₂ are each independently selected from the group consisting of hydrogen, halogen, C₁-C₈ perfluoroalkyl, C₁-C₈ alkyl, C₁-C₈ thioalkoxy and C₁-C₈ alkoxy; and
one of A₁ and A₂ is CH, and the other is N.

36. A method for preparing an oral solid dosage form comprising:
I) blending a carbamate compound of the following Formula 1, or a pharmaceutically acceptable salt, isomer, solvate or hydrate thereof as an active ingredient with excipients comprising a diluent, a lubricant, a binder and a disintegrant, and then compacting;
II) milling and sieving the mixture obtained in step (I) by screening through a sieve;
III) post-blending the granules sieved in step (II) with excipients comprising a disintegrant and a lubricant; and
IV) formulating the granules obtained by post-blending in step (III): 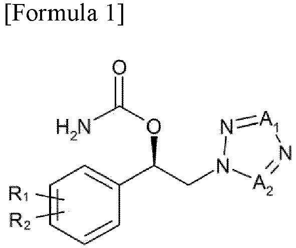 wherein,
R₁ and R₂ are each independently selected from the group consisting of hydrogen, halogen, C₁-C₈ perfluoroalkyl, C₁-C₈ alkyl, C₁-C₈ thioalkoxy and C₁-C₈ alkoxy; and
one of A₁ and A₂ is CH, and the other is N.

37. The method for preparing an oral solid dosage form according to Claim 35, wherein the active ingredient is micronized and has the particle diameter d(0.9) of 300 µm or less.

38. The method for preparing an oral solid dosage form according to Claim 34 or 36, wherein the active ingredient is not micronized and has the particle diameter d(0.9) of 300 µm to 1,300 µm.

39. The method for preparing an oral solid dosage form according to Claim 34, wherein a glidant is further mixed in step (iii).

40. The method for preparing an oral solid dosage form according to Claim 35, wherein a glidant is further mixed in step (c).

41. The method for preparing an oral solid dosage form according to any one of Claims 34 to 40, wherein the carbamate compound of Formula 1 is carbamic acid (R)-1-(2-chlorophenyl)-2-tetrazol-2-yl-ethyl ester of the following Formula 2:
